# EUROPÄISCHE PATENTANMELDUNG

(11) **EP 0 538 691 A1**
(43) Veröffentlichungstag der Anmeldung: **28.04.1993**
(21) Anmeldenummer: 92117262.3
(22) Anmeldetag: 09.10.1992
(51) Int. Cl.: C07D 333/38, C07D 333/48, A61K 31/385

(54) **Substituierte Tetrahydrothiophene, Verfahren zu ihrer Herstellung und ihre Verwendung als Arzneimittel**

(30) Priorität: 22.10.1991 DE 4134755
(71) Anmelder: BAYER AG, 51368 Leverkusen (DE)
(72) Erfinder: Mittendorf, Joachim, Dr., W-5600 Wuppertal (DE); Kunisch, Franz, Dr., W-5068 Odenthal-Glöbusch (DE); Plempel, Manfred, Dr., W-5657 Haan (DE)

(57) **Zusammenfassung**

Die vorliegende Erfindung betrifft substituierte Tetrahydrothiophene der allgemeinen Formel (I) 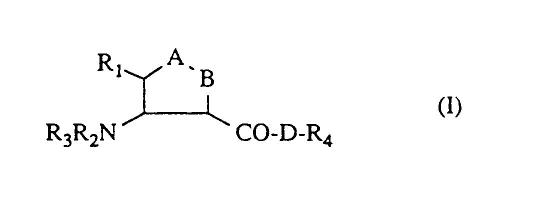
in welcher A, B, R₁, R₂, R₃, D und R₄ die in der Beschreibung angegebene Bedeutung haben, die Verwendung von teilweise bekannten substituierten Tetrahydrothiophenen als Arzneimittel, insbesondere als antimykotische Mittel, neue Wirkstoffe und Verfahren zu ihrer Herstellung.

## Beschreibung

Die Erfindung betrifft die Verwendung von teilweise bekannten substituierten Tetrahydrothiophenen als Arzneimittel, insbesondere als antimykotische Mittel, neue Wirkstoffe und Verfahren zu ihrer Herstellung.

Es ist bereits bekannt, daß 2,5-Diaryl-tetrahydrothiophene Antagonisten des Platelet aktivierenden Faktors sind [vgl. EP 365 089 A]. Außerdem wird in der Publikation J. Org. Chem. 12, (1947) 174, 180 die Verbindung (±)-trans-4-Ethoxycarbonylamino-tetrahydrothiophene-3-carbonsäure ohne pharmakologische Wirkung beschrieben.

Es wurde nun gefunden, daß die substituierten Tetrahydrothiophene der allgemeinen Formel (I), 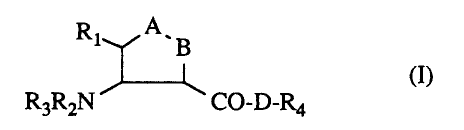
in welcher
- A und B: stets verschieden sind und
für ein Schwefelatom oder für die Gruppe der Formel -SO, -SO₂ oder -CHR⁵ stehen,
worin
- R⁵: Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
- R¹: für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR⁶R⁷ substituiert ist,
worin
- R⁶ und R⁷: gleich oder verschieden sind und Wasserstoff, Phenyl oder geradkettiges oder verzweigtes Alkyl mit bis zu 6 Kohlenstoffatomen bedeuten,
- R²: für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel -SO₂R⁸ steht,
worin
- R⁸: geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR⁶R⁷ substitituiert sind,
worin
- R⁶ und R⁷: die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR⁶R⁷ oder -SO₂R⁸ substituiert ist,
worin
- R⁶, R⁷ und R⁸: die oben angegebene Bedeutung haben,
- R³: für Wasserstoff oder für geradkettiges oder verzweigtes Alkyl mit bis zu 8 Kohlenstoffatomen steht, das gegebenenfalls durch Phenyl substituiert ist,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauerstoff- oder Schwefelatom oder für die 〉NH-Gruppe steht,
- R⁴: für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR⁶R⁷ oder -SO₂R⁸ substituiert sind,
worin
- R⁶, R⁷ und R⁸: die oben angegebene Bedeutung haben,
oder für den Fall, daß D für die 〉NH-Gruppe steht,
- R⁴: für die Gruppe der Formel -SO₂R⁸ steht, worin
- R⁸: die oben angegebene Bedeutung hat,
überraschenderweise eine starke antimikrobielle, insbesondere starke antimykotische Wirkung gegen Dermatophyten, Sproßpilze und biphasische Pilze zeigen und somit geeignet sind zur Verwendung bei der Bekämpfung der Dermatomykosen und Systemmykosen.

Auch die physiologisch unbedenklichen Säureadditions-Salze und Metallsalzkomplexe der Verbindungen der allgemeinen Formel (I) sowie die Racemformen, die Antipoden, die Diastereomerengemische und die einzelnen Isomere sind bevorzugt für diese Verwendung.

Bevorzugt verwendet werden Verbindungen der allgemeinen Formel (I), in welcher
- A und B: stets verschieden sind und
für ein Schwefelatom oder für die Gruppe der Formel -SO, -SO₂ oder -CHR⁵ stehen,
worin
- R⁵: Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
- R¹: für Wasserstoff oder für geradkettiges oder verzweigtes Alkyl mit bis zu 6 Kohlenstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ substituiert ist,
worin
- R⁶ und R⁷: gleich oder verschieden sind und Wasserstoff oder geradkettiges oder verzweigtes Alkyl mit bis zu 4 Kohlenstoffatomen bedeuten,
- R²: für Wasserstoff oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder
für Benzoyl steht, das gegebenenfalls wie oben beschrieben substituiert ist,
oder
für eine Gruppe der Formel -SO₂R⁸ steht,
worin
- R⁸: geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel -NR⁶R⁷ substituiert sind,
worin
- R⁶ und R⁷: die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel -NR⁶R⁷ oder -SO₂R⁸ substituiert ist,
worin
- R⁶, R⁷ und R⁸: die oben angegebene Bedeutung haben,
- R³: für Wasserstoff oder für geradkettiges oder verzweigtes Alkyl mit bis zu 6 Kohlenstoffatomen oder Benzyl steht,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauerstoff- oder Schwefelatom oder für die 〉NH-Gruppe steht,
- R⁴: für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ oder -SO₂R⁸ substituiert sind,
worin
- R⁶, R⁷ und R⁸: die oben angegebene Bedeutung haben,
oder für den Fall, daß D für die 〉NH-Gruppe steht,
- R⁴: für die Gruppe der Formel -SO₂R⁸ steht,
worin
- R⁸: die oben angegebene Bedeutung hat,
gegebenenfalls in einer isomeren Form und deren physiologisch unbedenkliche Säureadditions-Salze und Metallsalzkomplexe bei der Bekämpfung der Dermatomykosen und Systemmykosen.

Besonders bevorzugt verwendet werden Verbindungen der allgemeinen Formel (I),
in welcher
- A und B: stets verschieden sind und
für ein Schwefelatom oder für die Gruppe der Formel -SO, -SO₂ oder -CHR⁵ stehen,
worin
- R⁵: Wasserstoff oder geradkettiges oder verzweigtes Alkyl mit bis zu 4 Kohlenstoffatomen bedeutet,
- R¹: für Wasserstoff oder
für geradkettiges oder verzweigtes Alkyl mit bis zu 4 Kohlenstoffatomen steht,
- R²: für Wasserstoff oder
für geradkettiges oder verzweigtes Alkyl mit bis zu 4 Kohlenstoffatomen steht, oder
für geradkettiges oder verzweigtes Acyl mit bis zu 4 Kohlenstoffatomen oder
für eine Gruppe der Formel -SO₂R⁸ steht,
worin
- R⁸: geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
- R³: für Wasserstoff oder
für geradkettiges oder verzweigtes Alkyl mit bis zu 4 Kohlenstoffatomen steht,
oder
- R² und R³: gemeinsam für den Rest der Formel =CHR^{5'} stehen,
worin
- R^{5'}: die oben angegebene Bedeutung von R⁵ hat und mit dieser gleich oder verschieden ist,
- D: für ein Sauerstoff- oder ein Schwefelatom oder für die
〉NH-Gruppe steht,
- R⁴: für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel -NR⁶R⁷ oder -SO₂R⁸ substituiert sind,
worin
- R⁶und R⁷: gleich oder verschieden sind und Wasserstoff, Methyl oder Ethyl bedeuten
und
- R⁸: die oben angegebene Bedeutung hat,
oder im Fall, daß D für die 〉NH-Gruppe steht,
- R⁴: für die Gruppe der Formel -SO₂R⁸ steht,
worin
- R⁸: die oben angegebene Bedeutung hat,
gegebenenfalls in einer isomeren Form, und deren physiologisch unbedenkliche Säureadditions-Salze und Metallsalzkomplexe bei der Bekämpfung der Dermatomykosen und Systemmykosen.

Ganz besonders bevorzugt verwendet werden Verbindungen der allgemeinen Formel (I), in welcher die beiden Substituenten -NR²R³ und -CO-D-R⁴ in der cis-Stellung vorliegen bei der Bekämpfung der Dermatomykosen und Systemmykosen.

Die Erfindung betrifft außerdem neue Verbindungen der allgemeinen Formel (Ia) 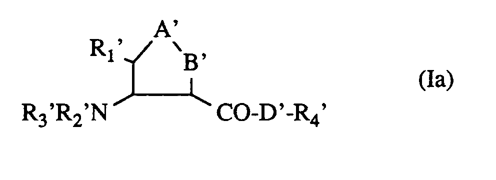
in welcher
- A' und B': stets verschieden sind und
für ein Schwefelatom oder für die Gruppe der Formel -SO, -SO₂ oder -CHR⁹ stehen,
worin
- R⁹: Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
- R^{1'}: für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR^{6'}R^{7'} substituiert ist,
worin
- R^{6'} und R^{7'}: gleich oder verschieden sind und Wasserstoff, Phenyl oder geradkettiges oder verzweigtes Alkyl mit bis zu 6 Kohlenstoffatomen bedeuten,
- R^{2'}: für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen,
oder
für Benzoyl steht, das gegebenenfalls wie oben beschrieben substituiert ist,
oder
für eine Gruppe der Formel -SO₂R^{8'} steht,
worin
- R^{8'}: geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR^{6'}R^{7'} substitituiert sind,
worin
- R^{6'} und R^{7'}: die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR^{6'}R^{7'} oder -SO₂R^{8'} substituiert ist,
worin
- R^{6'},R^{7'} und R^{8'}: die oben angegebene Bedeutung haben,
- R^{3'}: für Wasserstoff oder für geradkettiges oder verzweigtes Alkyl mit bis zu 8 Kohlenstoffatomen steht, das gegebenenfalls durch Phenyl substituiert ist,
oder
- R^{2'} und R^{3'}: gemeinsam für den Rest der Formel =CHR^{9'} stehen,
worin
- R^{9'}: die oben angegebene Bedeutung von R⁹ hat und mit dieser gleich oder verschieden ist,
- D': für ein Sauerstoff- oder Schwefelatom oder für die 〉NH-Gruppe steht,
- R^{4'}: für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR^{6'}R^{7'} oder -SO₂R^{8'} substituiert sind,
worin
- R^{6'}, R^{7'} und R^{8'}: die oben angegebene Bedeutung haben,
oder für den Fall, daß D für die 〉NH-Gruppe steht,
- R^{4'}: für die Gruppe der Formel -SO₂R^{8'} steht,
worin
- R^{8'}: die oben angegebene Bedeutung hat,
mit der Maßgabe, daß wenn A' für ein Schwefelatom, B' für die -CH₂-Gruppe, D' für ein Sauerstoffatom und R^{1'}, R^{2'}, R^{3'} und R^{4'} für Wasserstoff stehen, die beiden Substituenten -NR^{2'}R^{3'} und -CO-D'-R^{4'} nicht beide in trans-Stellung vorliegen dürfen.

Die erfindungsgemäßen Verbindungen der allgemeinen Formeln (I) und (Ia) können auch in Form ihrer Salze vorliegen. Im allgemeinen seien hier Salze mit organischen oder anorganischen Basen oder Säuren genannt.

Zu den Säuren, die addiert werden können, gehören vorzugsweise Halogenkohlenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Malonsäure, Oxalsäure, Gluconsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Physiologisch unbedenkliche Salze können ebenso Metall- oder Ammoniumsalze der erfindungsgemäßen Verbindungen sein, welche eine freie Carboxylgruppe besitzen, sein. Besonders bevorzugt sind z.B. Natrium-, Kalium-, Magnesium oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen der allgemeinen Formeln (I) und (Ia) können in stereoisomeren Formen existieren, beispielsweise entweder wie Bild und Spiegelbild (Enantiomere), oder die sich nicht wie Bild und Spiegelbild (Diastereomere) verhalten, beziehungsweise als Diastereomerengemisch oder als reine cis- oder trans-Isomere vorliegen. Die Erfindung betrifft sowohl die Antipoden, Racemformen, Diastereomerengemische sowie die reinen Isomeren. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962]. Die Trennung in die stereoisomer einheitlichen Verbindungen erfolgt beispielsweise über eine chromatographische Racematspaltung von diastereomeren Estern und Amiden oder an optisch aktiven Phasen. Außerdem ist eine Kristallisation von diastereomeren Salzen möglich.

Die erfindungsgemäßen Verbindungen der allgemeinen Formel (I) und die neuen Verbindungen der allgemeinen Formel (Ia) und ihre Säureadditions-Salze und Metallsalzkomplexe weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden:
Alle Dermatomykosen und Systemmykosen, insbesondere solche, die durch die oben genannten Erreger hervorgerufen werden.

Die Verbindungen der allgemeinen Formeln (I) und (Ia) können hergestellt werden, indem man

### [A] im Fall, daß A und A' jeweils entsprechend für die -CHR⁵ oder -CHR⁹-Gruppe und B bzw. B' für ein Schwefelatom stehen,

### Verbindungen der allgemeinen Formel (II)

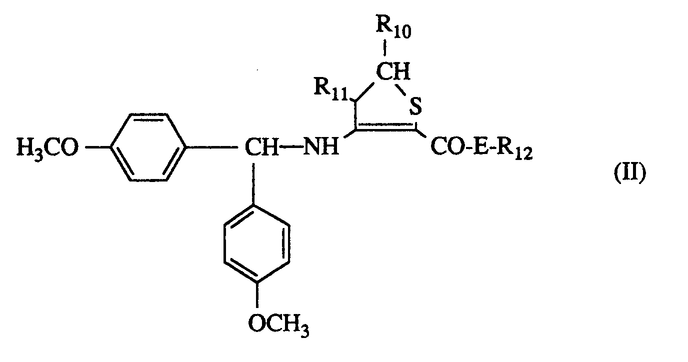

in welcher
- R¹⁰: die oben angegebene Bedeutung von R⁵ und R⁹ umfaßt,
- R¹¹: den oben angegebenen Bedeutungsumfang von R¹ und R^{1'} umfaßt,
- E: für ein Sauerstoffatom steht
und
- R¹²: für C₁-C₆-Alkyl steht,
zunächst mit Natriumcyanoborhydrid in inerten Lösemittel,
in die Verbindungen der allgemeinen Formel (III) 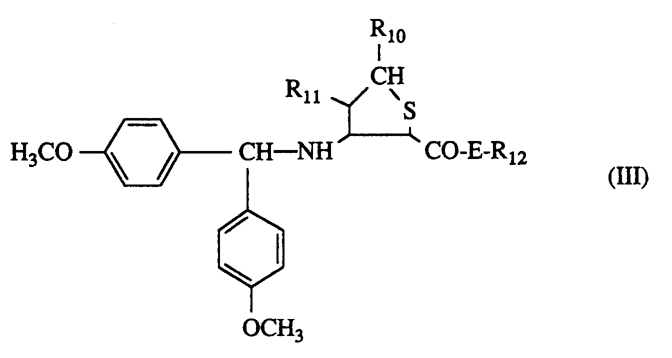
in welcher
- R¹⁰, R¹¹, R¹² und E: die oben angegebene Bedeutung haben
überführt,
und anschließend mit Säuren und Wasser, vorzugsweise Essigsäure, die Aminfunktion deblockiert
oder

### [B] im Fall, daß A und A' für ein Schwefelfatom und B bzw. B' für die -CHR⁵ oder -CHR⁹-Gruppe stehen,

### Verbindungen der allgemeinen Formel (IV)

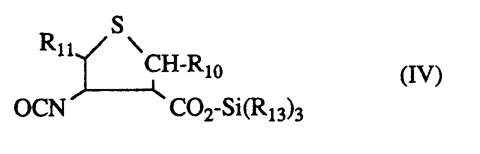

in welcher
- R¹⁰ und R¹¹: die oben angegebene Bedeutung haben
und
- R¹³: für einen C₁-C₃-Alkylrest steht,
in Ethern, vorzugsweise Diethylether, in Anwesenheit von Wasser
zunächst zu den Verbindungen der allgemeinen Formel (V) 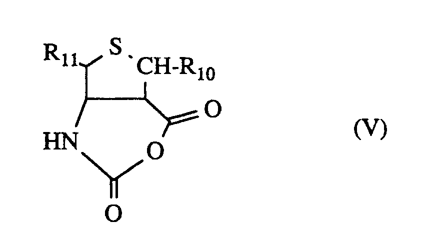
in welcher
- R¹⁰ und R¹¹: die oben angegebene Bedeutung haben,
umsetzt
und in einem nächsten Schritt mit Säuren, vorzugsweise Salzsäure und nachfolgend Propylenoxid, unter Ringöffnung, in die Verbindungen der allgemeinen Formel (Ib) 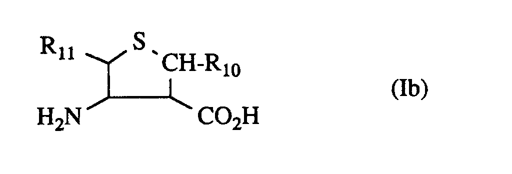
in welcher
- R¹⁰ und R¹¹: die oben angegebene Bedeutung haben,
überführt,
oder

### [C] im Fall, daß B und B' für die -SO oder -SO₂-Gruppe stehen,

### Verbindungen der allgemeinen Formel (Ic)

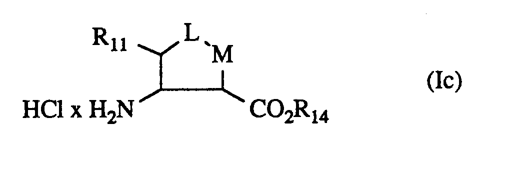

in welcher
- R¹⁰ und R¹¹: die oben angegebene Bedeutung haben,
- R¹⁴: für C₁-C₄-Alkyl steht
und
- L und M: verschieden sind und für ein Schwefelatom oder für die -CHR¹⁰-Gruppe stehen,
zunächst in inerten Lösemitteln, in Anwesenheit einer Base, vorzugsweise Triethylamin, nach Blockierung der freien Aminfunktion zu den Verbindungen der allgemeinen Formel (VI) 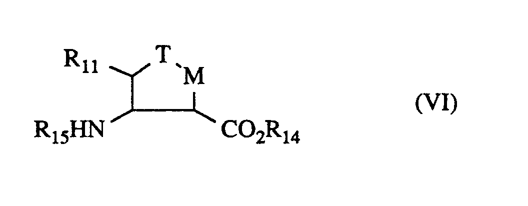
in welcher
- M und T: verschieden sind, und M die oben angegebene Bedeutung hat und T für die -SO oder -SO₂-Gruppe steht,
- R¹⁰, R¹¹ und R¹⁴: die oben angegebene Bedeutung haben
und
- R¹⁵: für eine literaturbekannte Aminoschutzgruppe, vorzugsweise tert.Butoxycarbonyl (BOC) steht
mit Oxidationsmitteln, vorzugsweise m-Chlorperbenzoesäure oxidiert und anschließend nach üblicher Methode, vorzugsweise mit Säuren, die Schutzgruppe abspaltet,
und im Fall der Säuren [(I), (Ia) D, E=O, R⁴/R^{4'}=H] gegebenenfalls die entsprechenden Ester verseift,
und im Fall der für D/A und R⁴/R^{4'} oben aufgeführten anderen Definitionen, ebenfalls nach üblichen Verfahren, wie beispielsweise Amidierung, Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung, derivatisiert.

Die erfindungsgemäßen Verfahren können durch folgendes Formelschema beispielhaft erläutert werden: 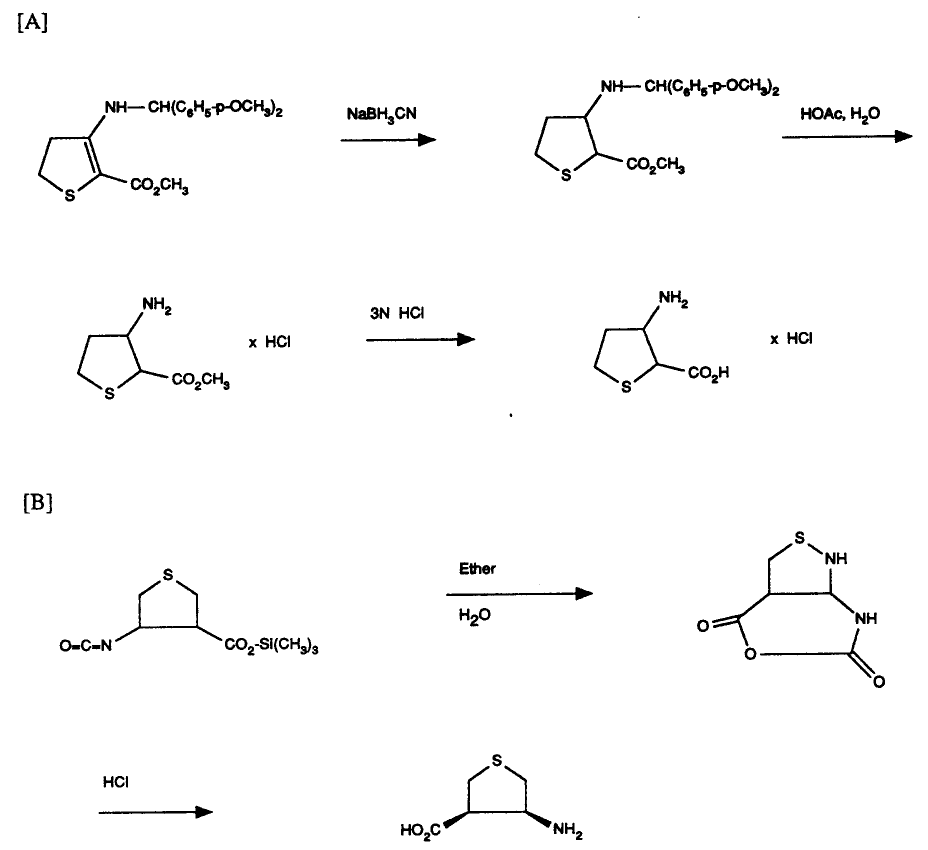 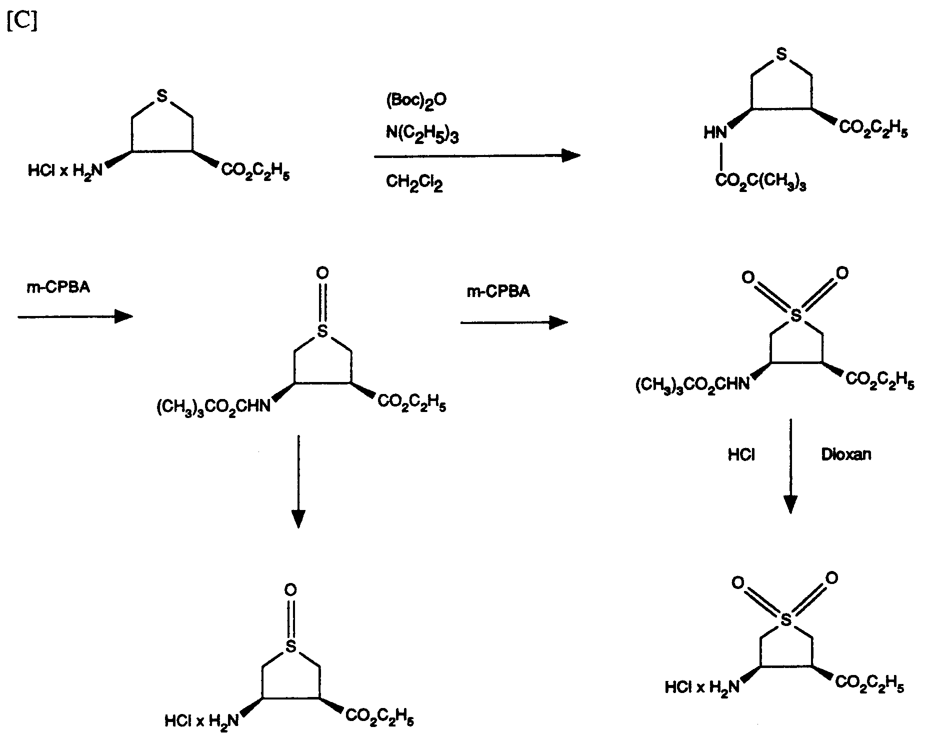
Als Lösemittel für die Verfahren [A], [B] und [C] kommen Wasser und alle inerten organischen Lösemittel in Frage, die sich unter den Reaktionsbedingungen nicht verändern. Hierzu gehören vorzugsweise Alkohole wie Methanol, Ethanol, Propanol, Isopropanol, Ether wie Diethylether, Dioxan, Tetrahydrofuran, Glykolmonomethylether oder Glykoldimethylether, oder Amide wie Dimethylformamid, Dimethylacetamid oder Hexamethylphosphorsäuretriamid, oder Eisessig, Dimethylsulfoxid, Acetonitril oder Pyridin. Bevorzugt sind für die einzelnen Schritte Diethylether, Dioxan, Methanol, Ethanol und Dichlormethan.

Die Reaktionstemperaturen können in einem größeren Breich varriiert werden. Im allgemeinen arbeitet man zwischen -78°C und +150°C, vorzugsweise zwischen -10°C und +100°C.

Die Umsetzungen können bei Normaldruck, aber auch bei erhöhtem oder erniedrigtem Druck (z.B. 0,5 bis 3 bar) durchgeführt werden. Im allgemeinen arbeitet man bei Normaldruck.

Bei der Durchführung der erfindungsgemäßen Verfahrensvarianten [A], [B] und [C] ist das Verhältnis der an der Reaktion beteiligten Stoffe beliebig. Im allgemeinen arbeitet man jedoch mit molaren Mengen der Reaktanden. Die Isolierung und Reinigung der erfindungsgmäßen Substanzen erfolgt vorzugsweise derart, daß man das Lösemittel im Vakuum abdestilliert und den gegebenenfalls erst nach Eiskühlung kristallin erhaltenen Rückstand aus einem geeigneten Lösemittel umkristallisiert. In einigen Fällen kann es erforderlich sein, die erfindungsgemäßen Verbindungen durch Chromatographie zu reinigen.

Als Oxidationsmittel eignen sich beispielsweise Natriumperjodat, Persäuren wie m-Chlorperbenzoesäure oder Kaliumpermanganat. Bevorzugt sind m-Chlorperbenzoesäure und Natriumperjodat.

Als Basen eignen sich organische Amine (Trialkyl(C1-C6)amine) wie beispielsweise Triethylamin oder Heterocyclen wie Pyridin, Methylpiperidin, Piperidin oder Morpholin. Bevorzugt ist Triethylamin.

Als Säuren für die Ringöffnung (V) werden im allgemeinen Mineralsäuren eingesetzt. Bevorzugt werden hierbei Chlorwasserstoffsäure, Bromwasserstoffsäure, Schwefelsäure, Phosphorsäure oder aber Gemische der genannten Säuren eingesetzt. Bevorzugt ist Chlorwasserstoffsäure.

Als Säuren für die Deblockierung (III) eignen sich C₁-C₆-Carbonsäuren wie beispielsweise Essigsäure oder Propionsäure. Bevorzugt ist Essigsäure.

Die Säure wird im allgemeinen in einer Menge von 2 mol bis 30 mol, bevorzugt von 5 mol bis 15 mol jeweils bezogen auf 1 mol der Verbindungen der allgemeinen Formeln (III) und (V) eingesetzt

Die Verseifung der Carbonsäureester erfolgt nach üblichen Methoden, indem man die Ester in inerten Lösemitteln mit üblichen Basen behandelt, wobei die zunächst entstehenden Salze durch Behandeln mit Säure in die freien Carbonsäuren überführt werden können.

Die Verseifung der Carbonsäureester kann ebenso mit einer der oben aufgeführten Säuren durchgeführt werden.

Als Basen eignen sich für die Verseifung die üblichen anorganischen Basen. Hierzu gehören bevorzugt Alkalihydroxide oder Erdalkalihydroxide wie beispielsweise Natriumhydroxid, Kaliumhydroxid oder Bariumhydroxid, oder Alkalicarbonate wie Natrium- oder Kaliumcarbonat oder Natriumhydrogencarbonat, oder Alkalialkoholate wie Natriumethanolat, Natriummethanolat. Kaliumethanolat, Kaliummethanolat oder Kalium- tert.butanolat. Besonders bevorzugt werden Natriumhydroxid oder Kaliumhydroxid eingesetzt.

Als Lösemittel eignen sich für die Verseifung Wasser oder die für eine Verseifung üblichen organischen Lösemittel. Hierzu gehören bevorzugt Alkohole wie Methanol, Ethanol, Propanol, Isopropanol oder Butanol, oder Ether wie Tetrahydrofuran oder Dioxan, oder Dimethylformamid oder Dimethylsulfoxid. Besonders bevorzugt werden Alkohole wie Methanol, Ethanol, Propanol oder Isopropanol verwendet. Ebenso ist es möglich, Gemische der genannten Lösemittel einzusetzen.

Die Verseifung wird im allgemeinen in einem Temperaturbereich von 0°C bis +100°C, bevorzugt von +20°C bis +80°C durchgeführt.

Im allgemeinen wird die Verseifung bei Normaldruck durchgeführt. Es ist aber auch möglich, bei Unterdruck oder bei Überdruck zu arbeiten (z.B. von 0,5 bis 5 bar).

Bei der Durchführung der Verseifung wird die Base oder die Säure im allgemeinen in einer Menge von 1 bis 3 mol, bevorzugt von 1 bis 1,5 mol bezogen auf 1 mol des Esters eingesetzt. Besonders bevorzugt verwendet man molare Mengen der Reaktanden.

Bei der Durchführung der Reaktion entstehen im ersten Schritt die Salze der erfindungsgemäßen Verbindungen als Zwischenprodukte, die isoliert werden können. Die erfindungsgemäßen Säuren erhält man durch Behandeln der Salze mit üblichen anorganischen Säuren. Hierzu gehören bevorzugt Mineralsäuren wie beispielsweise Chlorwasserstoffsäure, Bromwasserstoffsäure, Schwefelsäure oder Phosphorsäure. Es hat sich bei der Herstellung der Carbonsäuren als vorteilhaft erwiesen, die basische Reaktionsmischung der Verseifung in einem zweiten Schritt ohne Isolierung der Salze anzusäuern. Die Säuren können dann in üblicher Weise isoliert werden.

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.-Butoxycarbonyl, Allyloxycarbonyl, Vinyloxycarbonyl, 2-Nitrobenzyloxycarbonyl, 3,4,5-Trimethoxybenzyloxycarbonyl, Cyclohexylcarbonyl, 1,1-Dimethylethoxycarbonyl, Adamantylcarbonyl, Phthaloyl, 2,2,2-Trichlorethoxycarbonyl, 2,2,2-Trichlor-tert.-butoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl oder 4-Nitrophenyl.

Beispielhaft für die oben aufgeführten Derivatisierungsmöglichkeiten sollen hier die Amidierung und Sulfonierung bzw. Sulfoamidierung erläutert werden.

Die Amidierung erfolgt im allgemeinen in inerten Lösemitteln in Anwesenheit einer Base und eines Dehydratisierungsmittels.

Als Lösemittel eignen sich hierbei inerte organische Lösemittel, die sich unter den Reaktionsbedingungen nicht verändern. Hierzu gehören Halogenkohlenwasserstoffe wie Dichlormethan, Trichlormethan, Tetrachlormethan, 1,2-Dichlorethan, Trichlorethan, Tetrachlorethan, 1,2-Dichlorethylen oder Trichlorethylen, Kohlenwasserstoffe wie Benzol, Xylol, Toluol, Hexan, Cyclohexan oder Erdölfraktionen, Nitromethan, Dimethylformamid, Acetonitril oder Hexamethylphosphorsäuretriamid. Ebenso ist es möglich, Gemische der Lösemittel einzusetzen. Besonders bevorzugt ist Dichlormethan.

Als Basen für die Amidierung eignen sich die üblichen basischen Verbindungen. Hierzu gehören vorzugsweise Alkali- und Erdalkalihydroxide wie Lithiumihydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Amidierung wird im allgemeinen in einem Temperaturbereich von 0°C bis 150°C, bevorzugt bei 25°C bis 40°C, durchgeführt.

Die Amidierung wird im allgemeinen bei Normaldruck durchgeführt. Es ist aber auch möglich, das Verfahren bei Unterdruck oder bei Überdruck durchzuführen (z.B. in einem Bereich von 0,5 bis 5 bar).

Als Dehydratisierungsreagenzien eignen sich Carbodiimide wie beispielsweise Diisopropylcarbodiimid, Dicyclohexylcarbodiimid oder N-(3-Dimethylaminopropyl)-N'-ethylcarbodiimid-Hydrochlorid oder Carbonylverbindungen wie Carbonyldiimidazol oder 1,2-Oxazoliumverbindungen wie 2-Ethyl-5-phenyl-1,2-oxazolium-3-sulfonat oder Propanphosphorsäureanhydrid oder Isobutylchloroformat oder Benzotriazolyloxy-tris-(dimethylamino)phosphonium-hexafluorophosphat oder Phosphonsäurediphenylesteramid oder Methansulfonsäurechlorid, gegebenenfalls in Anwesenheit von Basen wie Triethylamin oder N-Ethylmorpholin oder N-Methylpiperidin oder Dicyclohexylcarbodiimid und N-Hydroxysuccinimid [vgl. J.C. Sheehan, S.L. Ledis, J. Am. Chem. Soc. 95, 875 (1973); F.E. Freeman et al., J. Biol. Chem. 225, 507 (1982) und N.B. Benoton, K. Kluroda, Int. Pept. Prot. Res. 13, 403 (1979), 17, 187 (1981)].

Die Sulfonierung oder Sulfoamidierung erfolgt in den oben erwähnten inerten Lösemitteln, gegebenenfalls unter Einsatz der ebenfalls oben aufgeführten Basen und Dehydratisierungsmittel.

Die Sulfonierung und Sulfoamidierung werden im allgemeinen bei Normaldruck durchgeführt. Es ist aber auch möglich, die Verfahren bei Unterdruck oder Überdruck durchzuführen (z.B. in einem Bereich von 0,5 bis 5 bar).

Die Sulfonierung und die Sulfoamidierung werden im allgemeinen in einem Temperaturbereich von 0°C bis +150°C, bevorzugt von +25°C bis +40°C, durchgeführt.

Zur Amidierung eignen sich im allgemeinen die literaturbekannten, käuflichen Amine und deren Derivate [vgl. Houben-Weyl, "Methoden der organischen Chemie", Band XI/1 und XI/2].

Die Sulfonierung und Sulfoamidierung erfolgen im allgemeinen ebenfalls mit den üblichen Sulfonsäuren und deren aktivierte Derivate [vgl. Houben-Weyl, "Methoden der organischen Chemie", Band IX, S 407 ff; Beilstein 11, 26].

Die Veresterung der Säuren erfolgt nach üblicher Methode, indem man die Säuren gegebenenfalls in einem der oben aufgeführten Lösemittel in Anwesenheit eines Katalysators mit den entsprechenden Alkoholen umsetzt. Bevorzugt wird dieser Alkohol auch als Lösemittel eingesetzt.

Als Katalysatoren können anorganische Säuren, wie beispielsweise Schwefelsäure oder anorganische Säurechloride, wie beispielsweise Thionylchlorid, eingesetzt werden.

Im allgemeinen setzt man 0,01 bis 1, bevorzugt 0,05 bis 0,5 mol Katalysator bezogen auf 1 mol Reaktionspartner ein.

Sowohl die Veresterung als auch die Amidierung können gegebenenfalls über aktivierte Stufen der Carbonsäuren, wie beispielsweise Säurehalogenide, die aus der entsprechenden Säure durch Umsetzung mit Thionylchlorid, Phosphortrichlorid, Phosphorpentachlorid, Phosphortribromid oder Oxalylchlorid hergestellt werden können, verlaufen.

Die Säureadditions-Salze der Verbindungen der Formel (I) können in einfacher Weise nach üblichen Salzbildungsmethoden, z.B. durch Lösen einer Verbindung der Formel (I) in einem geeigneten Lösemittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösemittel gereinigt werden.

Die Verbindungen der allgemeinen Formel (II) sind neu und können hergestellt werden, indem man

### Verbindungen der allgemeinen Formel (VII)

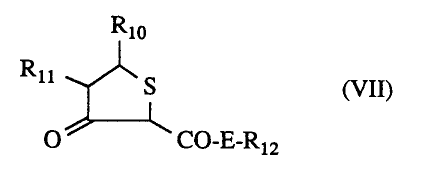

in welcher
- R¹⁰, R¹¹, R¹² und E: die oben angegebene Bedeutung haben,
mit 4,4'-Dimethoxy-benzhydrylamin, in Anwesenheit von p-Toluolsulfonsäure in einem der oben aufgeführten Lösemittel, vorzugsweise Benzol umsetzt.

Die Verbindungen der allgemeinen Formel (VII) sind teilweise bekannt oder neu und können dann aber in Analogie zu literaturbekannten Verfahren hergestellt werden.

Die Verbindungen der allgemeinen Formel (III) sind neu und können nach dem oben angegebenen Verfahren hergestellt werden.

Die Verbindungen der allgemeinen Formel (IV) sind neu und können hergestellt werden, indem man

### Verbindungen der allgemeinen Formel (VIII)

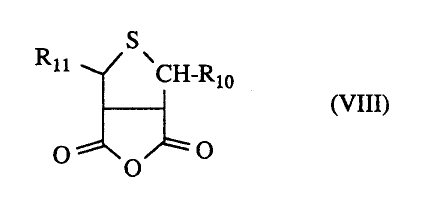

in welcher
- R¹⁰ und R¹¹: die oben angegebene Bedeutung haben,

mit Verbindungen der allgemeinen Formel (IX)

(R¹³)₄SiN₃ (IX)

in welcher
- R¹³: die oben angegebene Bedeutung hat,
in einem Temperaturbereich von +20°C bis +80°C, vorzugsweise bei 80°C, umsetzt.

Die Verbindungen der allgemeinen Formel (VIII) sind teilweise bekannt, insbesondere das unsubstituierte cis-Isomere, oder neu und können dann aber in Analogie zu bekannten Verfahren über die entsprechenden Dicarbonsäuren durch Dehydratisierung hergestellt werden [vgl. J. Org. Chem. 12, 174 (1947)].

Die Verbindungen der allgemeinen Formel (IX) sind bekannt [vgl. Fieser 1, 1236; 3, 316; 5, 719; 6, 632; 7, 394; 9, 21; 10, 14].

Die Verbindungen der allgemeinen Formel (V) sind neu und können nach dem oben aufgeführten Verfahren hergestellt werden.

Die Verbindungen der allgemeinen Formel (Ib) sind mit Ausnahme des cis-Isomeren [vgl. J. Org. Chem. 12, 174 (1947)] neu und können wie unter [B] beschrieben hergestellt werden.

Die Verbindungen der allgemeinen Formel (VI) sind teilweise bekannt [vgl. Bull. Chem. Soc. Jpn. 40 (11), 2636-40; Chem. Ber. 123 (1990), 1990-2014], wobei gegebenenfalls andere Estergruppierungen vorliegen, oder neu und können dann aber nach der in der oben aufgeführten Literaturstelle vorgestellten Methode hergestellt werden.

Die Verbindungen der allgemeinen Formel (Ic) sind neu und können nach dem oben aufgeführten Verfahren hergestellt werden.

Die vorstehenden Herstellungsverfahren sind lediglich zur Verdeutlichung angegeben. Die Herstellung der erfindungsgemäßen Verbindungen der allgemeinen Formeln (I) und (Ia) ist nicht auf diese Verfahren beschränkt, jede Modifikation dieser Verfahren ist in gleicher Weise für die Herstellung anwendbar.

Die erfindungsgemäßen Verbindungen der allgemeinen Formeln (I) und (Ia) und ihre Säureadditions-Salze weisen antimikrobielle, insbesondere starke antimykotische Wirkungen auf. Sie besitzen ein sehr breites antimykotisches Wirkspektrum, insbesondere gegen Dermatophyten und Sproßpilze sowie biphasische Pilze, z.B. gegen Candida-Arten wie Candida albicans, Epidermophyton-Arten wie Epidermophyton floccosum, Aspergillus-Arten wie Aspergillus niger und Aspergillus fumigatus, Trichophyton-Arten wie Trichophyton mentagrophytes, Microsporon-Arten wie Microsporon felineum sowie Torulopsis-Arten, wie Torulopsis glabrata. Die Aufzählung dieser Mikroorganismen stellt keinesfalls eine Beschränkung der bekämpfbaren Keime dar, sondern hat nur erläuternden Charakter.

Als Indikationsbeispiele in der Humanmedizin können beispielsweise genannt werden:
Dermatomykosen und Systemmykosen durch Trichophyton mentagrophytes und andere Trichophytonarten, Microsporonarten sowie Epidermophyton floccosum, Sproßpilze und biphasische Pilze sowie Schimmelpilze hervorgerufen.

Als Indikationsgebiete in der Tiermedizin können beispielsweise aufgeführt werden: Alle Dermatomykosen und Systemmykosen, insbesondere solche, die durch die oben genannten Erreger hervorgerufen werden.

Die erfindungsgemäßen Verbindungen wurden in den Applikationsarten i.v., s.c. und orale Gabe am Modell der Mäuse-Candidose auf ihre antimykotische in vivo-Wirksamkeit geprüft:
Männliche CF₁-SPF-Mäuse wurden mit 1-3 x 10⁶ Sprosszellen von C. albicans pro Tier durch Injektion der Keimsuspension in phys. NaCl-Lösung (0,2 ml/Tier) in die Schwanzvene infiziert. Nicht behandelte Kontrolltiere entwickeln unter diesen Infektionsbedingungen eine Nierencandidose und sterben zu 95-100% der eingesetzten Tiere innerhalb 6 Tagen p.i. an dieser Infektion. Wurden infizierte Tiere täglich 2 mal, beginnend mit dem Tag der Infektion, mit den erfindungsgemäßen Verbindungen oral oder parenteral in Dosen 2 x 25 bis 2 x 50 mg/kg Körpergewicht über 2 - 5 Tage behandelt, so überleben 60-90% der Tiere die Infektion in gutem Zustand. Die C.Yalbicans-Keimzahlen in den Nieren der infizierten und behandelten Tiere liegen am 4. Tag p.i. durchschnittlich um 2 Zehnerpotenzen unter denen von unbehandelten, infizierten Kontrolltieren.

In der folgenden Tabelle sind die in vivo-Wirkungen einiger erfindungsgemäßer Verbindungen im Modell der Mäusecandidose dargestellt:

**Tabelle [A]**

| Beispiel Nr. | Dosis mg/kg | Applikationsart | Zahl überleb. Tiere am 6. Tag p.i. |
|---|---|---|---|
| Kontrolle | - | - | 1/10 |
| 2 | 25 | sc,iv | 7/10 |
| 3 | 25 | sc,oral | 9/10 |
| 9 | 25 | sc | 7/10 |
| 11 | 25 | sc | 8/10 |

Die Verbindungen sind unter konventionellen Testbedingungen - Reihenverdünnungstest und Agardiffusionstest - in vitro nicht antimykotisch wirksam.

Die neuen Wirkstoffe können in bekannter Weise in die üblichen Formulierungen überführt werden, wie Tabletten, Dragees, Pillen, Granulate, Aerosole, Sirupe, Emulsionen, Suspensionen und Lösungen, unter Verwendung inerter, nicht-toxischer, pharmazeutisch geeigneter Trägerstoffe oder Lösemittel. Hierbei soll die therapeutisch wirksame Verbindung jeweils in einer Konzentration von etwa 0,5 bis 90-Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösemitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösemittel als Hilfslösemittel verwendet werden können.

Die Applikation erfolgt in üblicher Weise, vorzugsweise oral oder parenteral, insbesondere perlingual oder intravenös.

Für den Fall der parenteralen Anwendung können Lösungen des Wirkstoffs unter Verwendung geeigneter flüssiger Trägermaterialien eingesetzt werden.

Im allgemeinen hat es sich als vorteilhaft erwiesen, bei intravenöser Applikation Mengen von etwa 0,001 bis 10 mg/kg, vorzugsweise etwa 0,01 bis 5 mg/kg Körpergewicht zur Erzielung wirksamer Ergebnisse zu verabreichen, und bei oraler Applikation beträgt die Dosierung etwa 0,01 bis 25 mg/kg, vorzugsweise 0,1 bis 10 mg/kg Köpergewicht.

Trotzdem kann es gegebenenfalls erforderlich sein, von den genannten Mengen abzuweichen, und zwar in Abhängigkeit vom Körpergewicht bzw. der Art des Applikationsweges, vom individuellen Verhalten gegenüber dem Medikament, der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannnte obere Grenze überschrittten werden muß. Im Falle der Applikation größerer Mengen kann es empfehlenswert sein, diese in mehreren Einzelgaben über den Tag zu verteilen.

### Ausgangsverbindungen

### Beispiel I

### 3,4-cis-4-Isocyanato-tetrahydro-3-thiophencarbonsäure-trimethylsilylester

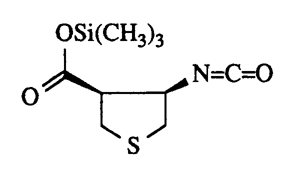

Eine Lösung von Thiophan-3,4-cis-dicarboxylat-anhydrid (10,0 g; 63,2 mmol) und Trimethylsilylazid (8,29 g, 72,0 mmol) in 60 ml Dioxan wird 2 h auf 80°C erhitzt. Das Solvens wird im Vakuum abgezogen und der Rückstand am Kugelrohr destilliert.
Ausbeute: 9,30 g (63% der Theorie)
Sdp.: 160-170°C/0,4 Torr
C₈H₁₅NO₃SSi (233,36)
¹H-NMR (CDCl₃): δ = 0,33 (s, 9H); 2,93 - 3,28 (m, 5H); 4,53 - 4,59 (m, 1H).

### Beispiel II

### 4a,5,7,7a-Tetrahydro-thieno[3,4-d]oxazin-2,4(1H)-dion

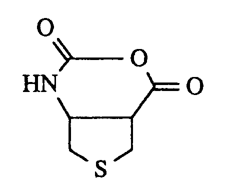

Eine Lösung der Verbindung aus Beispiel I (7,30 g, 31,3 mmol) in 45 ml Diethylether wird mit Wasser (0,28 g, 15,6 mmol) versetzt und 2 h bei 6°C stehen gelassen. Ausgefallenes Produkt wird abfiltriert und mit Diethylether gewaschen.
Ausbeute: 3,30 g (62% der Theorie)
C₆H₁₇NO₃S (173,2)
IR (KBr) max: 1811, 1727 cm⁻¹

### Beispiel III

### 3,4-cis-4-N-(tert.Butyloxycarbonyl)amino-tetrahydro-3-thiophen-carbonsäureethylester

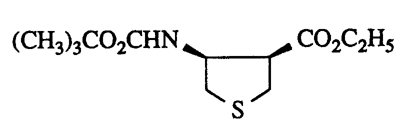

Eine Lösung der Verbindung aus Beispiel II(5,0 g, 24 mmol) und Triethylamin (7,1 g, 72 mmol) in 60 ml Dichlormethan wird mit Di-tert.Butyldicarbonat (7,9 g, 36 mmol) versetzt und 20 h bei Raumtemperatur gerührt. Das Solvens wird im Vakuum abgezogen und der Rückstand wird an Kieselgel chromatographiert (Ether/Petrolether = 1:2).
Ausbeute: 5,9 g (95% der Theorie)
C₁₂H₂₁NO₄S (259,4)
Smp.: 67°C

### Beispiel IV

### 3,4-cis-4-N-(tert.Butyloxycarbonyl)amino-tetrahydro-3-thiophen-1-oxidcarbonsäureethylester

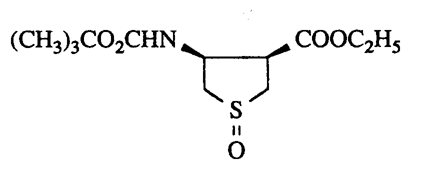

Zur Lösung aus Beispiel III (3,30 g, 12,7 mmol) in 60 ml Dichlormethan wird bei -78°C eine Lösung von m-Chlorperbenzoesäure (3,10 g, 12,7 mmol) in 30 mi Dichlormethan getropft. Man läßt auf 0°C erwärmen, versetzt unter Rühren mit 150 ml einer 10%igen wäßrigen Natriumbisulfitlösung und trennt die Phasen. Die organische Phase wird zweimal mit gesättigter wäßriger NaHCO₃-Lösung gewaschen und über Natriumsulfat getrocknet. Das Lösemittel wird im Vakuum abgezogen.
Ausbeute: 2,70g (77% der Theorie)
C₁₂H₂₁NO₅S (291,4)
Smp.: 115-120°C
Diastereomerenverhältnis D₁:D₂ = 2,2:1

### Beispiel V

### 3,4-cis-4-N-(tert.Butyloxycarbonyl)amino-tetrahydro-3-thiophen-1,1-dioxidcarbonsäureethylester

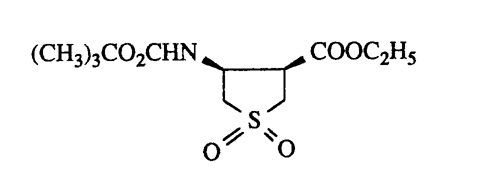

Zur Lösung der Verbindung aus Beispiel III (1,20 g, 4,1 mmol) in 20 ml Dichlormethan gibt man bei 0°C m-Chlorperbenzoesäure (1,00 g, 4,1 mmol) und läßt 5 h bei Raumtemperatur rühren. Man versetzt unter Rühren mit 20 ml einer 20%igen wäßrigen Natriumbisulfitlösung und trennt die Phasen. Die organische Phase wird zweimal mit gesättigter NaHCO₃-Lösung gewaschen und über Natriumsulfat getrocknet. Das Lösemittel wird im Vakuum abgezogen und der Rückstand aus Essigester umkristallisiert.
Ausbeute: 0,93 g (74% der Theorie)
C₁₂H₂₁NO₆S (307,4)
Smp.: 128°C

### Beispiel VI

### 3-N-(4,4'-Dimethoxybenzhydryl)amino-4,5-dihydro-thiophen-2-carbonsäuremethylester

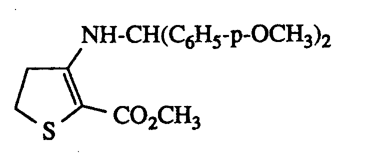

Eine Lösung von 4,5 g (28,0 mmol) Tetrahydrothiophen-3-on-2-carbonsäuremethylester, 6,9 g (28 mmol) 4,4'-Dimethoxy-benzhydrylamin und 0,1 g p-Toluolsulfonsäure werden in 50 mi Benzol 24 h am Wasserabscheider unter Rückfluß erhitzt. Anschließend wird mit 40 ml Toluol verdünnt, mit 40 ml 1%iger wäßriger NaHCO₃-Lösung und zweimal mit je 30 ml Wasser gewaschen. Die organische Phase wird über Na₂SO₄ getrocknet und das Lösemittel im Vakuum abgezogen. Der Rückstand wird an Kieselgel chromatographiert (Ether/Petrolether = 1:2)
Ausbeute: 7,58 g (70% der Theorie)
C₂₁H₂₃NO₄S (385,4)
R_{f} = 0,42 (Ether: Petrolether = 1:2)

### Beispiel VII

### 3-N-(4,4'-Dimethoxybenzhydryl)amino-tetrahydro-thiophen-2-carbonsäuremethylester

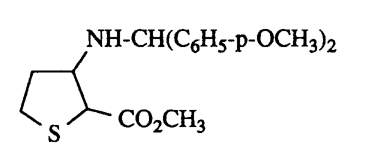

Eine Lösung von 0,50 g (1,30 mmol) der Verbindung aus Beispiel VI in 10 ml Ethanol wird mit 0.080 g (1,30 mmol) Natriumcyanoborhydrid und anschließend mit 6 N HCl versetzt bis der Indikator von Bromkresolgrün nach gelb umschlägt. Es wird 24 h bei Raumtemperatur gerührt, das Solvens im Vakuum abgezogen und der Rückstand mit 10 ml Essigester versetzt Die Lösung wird mit 10 ml 1%iger wäßriger NaHCO₃-Lösung und mit 10 ml Wasser gewaschen und über Na₂SO₄ getrocknet. Der Rückstand wird an Kieselgel chromatographiert (Ether: Petrolether = 1:2)
Ausbeute: 0,32 g (64% der Theorie)
C₂₁H₂₅NO₄S (387,5)
R_{f} = 0,34 (Ether: Petrolether = 1:2)

### Beispiel VIII und IX

### 3-N-(tert.-Butyloxycarbonyl)amino-tetrahydro-2-thiophen-carbonsäuremethylester

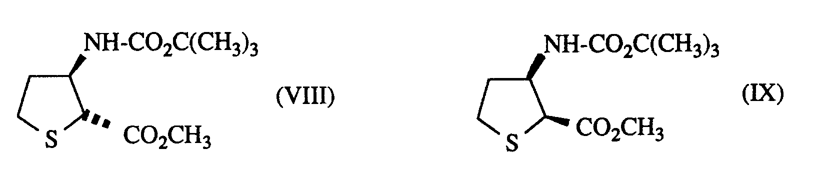

Eine Lösung aus Beispiel 8 (9,45 g; 58,6 mmol) in 150 mi Dichlormethan wird mit Triethylamin (17,3 g; 176 mmol) und anschließend mit Di-tert.-butyl-dicarbonat (19,3 g; 87,9 mmol) versetzt und 20 h bei Raumtemperatur gerührt. Das Lösungsmittel wird im Vakuum abgezogen und der Rückstand an Kieselgel chromatographiert (Ether/Petrolether = 1:2).
Diastereomer (VIII):
Ausbeute: 8,76 g (57 %)
R_{f} = 0.43 (Ether/Petrolether = 1:2), Smp.: 93°C
Diastereomer (IX):
Ausbeute: 4,08 g (19 %)
R_{f} = 0.35 (Ether/Petrolether = 1:2), Smp.: 69°C
C₁₁H₉NO₄S (261,34)

### Beispiel X

### 3-N-(tert.Butyloxycarbonyl)amino-tetrahydro-2-thiophen-1-oxid-carbonsäuremethylester

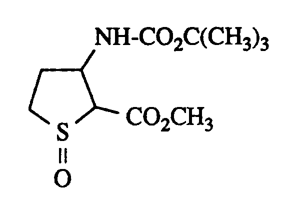

Zur Lösung aus Beispiel VIII (3,0 g; 11,4 mmol) in 60 ml Dichlormethan wird bei -78°C eine Lösung von m-Chlorperbenzoesäure (2,80 g; 11,4 mmol) in 30 ml Dichlormethan getropft. Man läßt auf 0°C erwärmen, versetzt unter Rühren mit 150 ml einer 10 %igen Natriumbisulfitlösung und trennt die Phasen. Die organische Phase wird zweimal mit gesättigter wäßriger NaHCO₃-Lösung gewaschen und über Na₂SO₄ getrocknet. Das Lösungsmittel wird im Vakuum abgezogen.
Ausbeute: 2,50 g (79 % eines Diastereomerengemisches)
C₁₁H₁₉NO₅S (277.3)
Smp.: 110-115°C

### Beispiel XI

### 3-N-(tert.-Butyloxycarbonyl)amino-tetrahydro-2-thiophen-1,1-dioxidcarbonsäuremethylester

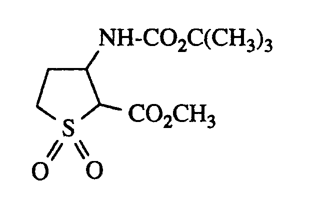

Zur Lösung aus Beispiel VIII (3,0 g; 11,4 mmol) in 90 ml CH₂Cl₂ gibt man bei -50°C m-Chlorperbenzoesäure (5,60 g; 22,8 mmol), läßt auf Raumtemperatur erwärmen und noch 5 h bei dieser Temperatur rühren. Man versetzt unter Rühren mit 150 ml einer 10 %igen Natriumbisulfitlösung und trennt die Phasen. Die organische Phase wird zweimal mit gesättigter wäßriger NaHCO₃-Lösung gewaschen und über Na₂SO₄ getrocknet. Das Lösungsmittel wird im Vakuum abgezogen.
Ausbeute: 2,40 g (72 % eines Diastereomerengemisches)
C₁₁H₁₉NO₆S (293.3)
Smp.: 95-98°C

### Herstellungsbeispiele

### Beispiel 1

### 3,4-cis-4-Amino-tetrahydro-3-thiophen-carbonsäureethylester-Hydrochlorid

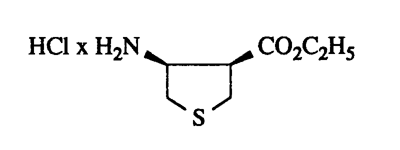

Eine Lösung der Verbindung aus Beispiel II (3,00 g, 17,3 mmol) in 35 ml Ethanol wird bei -10°-0°C mit Acetylchlorid (2,20 g, 28,0 mmol) versetzt, 20 h bei Raumtemperatur gerührt, im Vakuum auf ein Volumen von ca. 8 ml eingeengt, mit 6 ml Ether versetzt und ausgefallenes Produkt abfiltriert.
Ausbeute: 2,60 g (72% der Theorie)
C₇H₁₃NO₂S x HCl
Schmp.: 100°C

### Beispiel 2

### 3,4-cis-4-Amino-tetrahydro-3-thiophencarbonsäure-Hydrochlorid

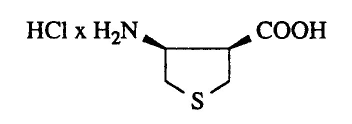

Eine Lösung der Verbindung aus Beispiel 1 (0,800 g, 3,80 mmol) in 55 ml 20%iger wäßriger HCl-Lösung wird 2 h unter Rückfluß erhitzt und anschließend im Vakuum zur Trockene eingedampft.
Ausbeute: 0,67 g (97% der Theorie)
C₅H₉NO₂S x HCl
Schmp.: 205-210°C

### Beispiel 3

### 3,4-cis-4-Amino-tetrahydro-3-thiophen-carbonsäuremethylester-Hydrochlorid

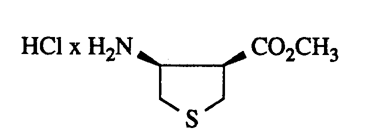

Eine Lösung der Verbindung aus Beispiel II (0,60 g, 3,46 mmol) in 10 ml Methanol wird bei -10°C-0°C mit Acetylchlorid (0,44 g, 5,60 mmol) versetzt, 20 h bei Raumtemperatur gerührt, im Vakuum auf ein Volumen von ca. 2 ml eingeengt und mit 3 ml Ether versetzt. Ausgefallenes Produkt wird abfiltriert.
Ausbeute: 0,30 g (46% der Theorie)
C₆H₁₁NO₂S x HCl
Smp.: 168°C

### Beispiel 4

### 3,4-cis-4-Amino-tetrahydro-3-thiophen-1-oxid-carbonsäureethylester-Hydrochlorid

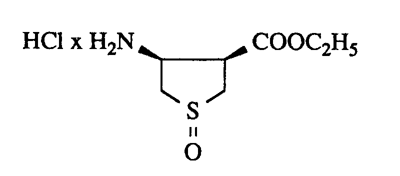

Eine Lösung der Verbindung aus Beispiel IV (2,00 g, 7,26 mmol) in 10 ml einer 4 N Lösung von HCl in 1,4-Dioxan wird 5 h bei Raumtemperatur gerührt. Ausgefallenes Produkt wird abgesaugt, mit Ether gewaschen und im Vakuum getrocknet.
Ausbeute: 0,71 g (44% der Theorie)
C₇H₁₃NO₃S x HCl
Smp.: 98°C
Diastereomerenverhältnis D₁:D₂ = 2,2:1

### Beispiel 5

### 3,4-cis-4-Amino-tetrahydro-3-thiophen-1-oxid-carbonsäure-Hydrochlorid

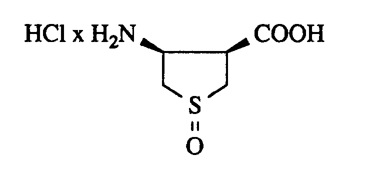

Eine Lösung der Verbindung aus Beispiel 4 (0,35 g, 1,54 mmol) in 30 ml 3 N HCl wird 2 h unter Rückfluß erhitzt. Die Lösung wird im Vakuum eingeengt und der Rückstand im Vakuum bei 50°C /0,1 Torr getrocknet.
Ausbeute: 0,30 g (96% der Theorie)
C₅H₉NO₃S x HCl
MS (FAB): m/z = 164 (M+H)⁺
Diastereomerenverhältnis D₁:D₂ = 2,2:1

### Beispiel 6

### 3,4-cis-4-Amino-tetrahydro-3-thiophen-1,1-dioxid-carbonsäureethylester-Hydrochlorid

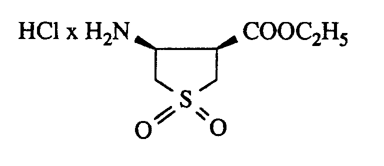

Eine Lösung der Verbindung aus Beispiel V (1,00 g, 3,30 mmol) in 5 ml 4 N HCl in Dioxan wird 3 h bei Raumtemperatur gerührt. Das Lösemittel wird im Vakuum abgezogen und der Rückstand im Vakuum bei 0,1 Torr / 50°C getrocknet.
Aubeute: 0,80g (100% der Theorie)
C₇H₁₃NO₄S x HCl
Smp.: 150-155°C

### Beispiel 7

### 3,4-cis-4-Amino-tetrahydro-3-thiophen-1,1-dioxid-carbonsäure-Hydrochlorid

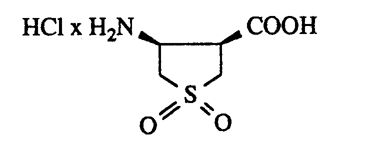

Eine Lösung der Verbindung aus Beispiel 6(0,50 g, 2,0 mmol) wird in 20 ml 3 N HCl 2 h unter Rückfluß erhitzt. Die Lösung wird in Vakuum eingeengt und der Rückstand im Vakuum bei 50°C/0,1 Torr getrocknet.
Ausbeute: 0,44g (100% der Theorie)
C₅H₉NO₄S x HCl
Smp.: 212°C

### Beispiel 8

### 3-Amino-tetrahydrothiophen-2-carbonsäuremethylester Hydrochlorid

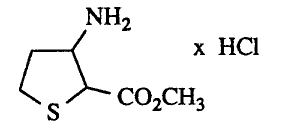

Eine Lösung von 0,48 g (1,24 mmol) der Verbindung aus Beispiel VII in 10 ml Essigsäure/Wasser (1:1) wird 5 min auf 80°C erhitzt. Man verdünnt mit 20 ml Wasser und extrahiert 2 mal mit je 10 ml Ether. Die wäßrige Phase wird im Vakuum bei 20°C eingeengt, der Rückstand wird in 5 ml Wasser aufgenommen und mit konz. NH₃ bis pH 10 versetzt. Es wird dreimal mit je 10 ml Ether extrahiert, die Etherphasen werden über Na₂SO₄ getrocknet und das Solvens wird im Vakuum abgezogen. Man versetzt mit 1 ml 2 N methanolischer HCl und zieht das Lösemittel im Vakuum ab. Der Rückstand wird im Vakuum bei 0,1 Torr / 50°C getrocknet
Ausbeute: 0,124 g (51% der Theorie)
C₆H₁₁NO₂S x HCl
R_{f} = 0,45 (Ether: Acetonitril : konz. NH₃ = 10:1:0,1)
Diastereomerenverhältnis 3:1

### Beispiele 9 und 10

### 3-Amino-tetrahydrothiophen-2-carbonsäuremethylester-Hydrochlorid

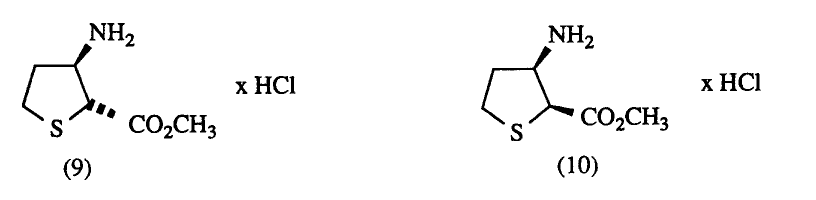

Eine Lösung von 1,50 g (5,74 mmol) der Verbindungen aus den Beispielen VIII und IX in 9 ml 4 N HCl in Dioxan wird 3 Stunden bei Raumtemperatur gerührt. Ausgefallenes Produkt wird abgesaugt, mit Ether gewaschen und getrocknet,
Diastereomer (9):
Ausbeute: 1,14 g (100 %)
Smp.: 146°C
C₆H₁₁NO₂S x HCl
Diastereomer (10):
Ausbeute: 0,99 g (87 %)
Smp.: 172°C
C₆H₁₁NO₂S x HCl

### Beispiele 11 und 12

### 3-Amino-tetrahydrothiophen-2-carbonsäure Hydrochlorid

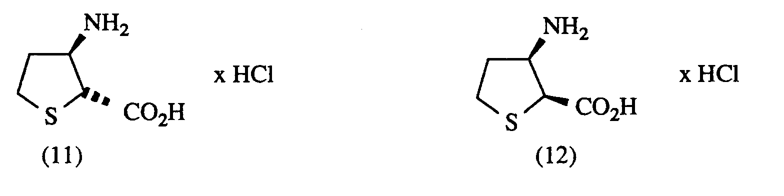

Eine Lösung von 0,50 g (2,5 mmol) der Verbindungen aus den Beispielen 9 und 10 in 25 mi 3 N HCl wird 3 h unter Rückfluß erhitzt. Das Solvens wird im Vakuum abgezogen und der Rückstand bei 0,1 Torr / 50°C getrocknet.
Diastereomer (11):
Ausbeute: 0,41 g (89 %)
Smp.: 231°C
Diastereomer (12):
Ausbeute: 0,42 g (91 %)
Smp.: 160°C
C₅H₉NO₂S x HCl

### Beispiel 13

### 3-Amino-tetrahydrothiophen-1,1-dioxid-2-carbonsäuremethylester Hydrochlorid

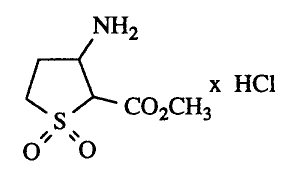

Eine Lösung aus Beispiel XI (3,50 g; 12,0 mmol) in 20 ml 4 N HCl in Dioxan wird 3 h bei Raumtemperatur gerührt. Das Lösungsmittel wird im Vakuum abgezogen und der Rückstand mit Tetrahydrofuran gewaschen.
Ausbeute: 1,46 g (77 % eines Diastereomerengemisches)
C₆H₁₁NO₄S x HCl (193.2 x 36.5)
Smp.: >250°C

### Beispiel 14

### 3-Amino-tetrahydrothiophen-1,1-dioxid-2-carbonsäure Hydrochlorid

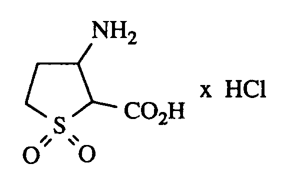

Eine Lösung aus Beispiel 13 (1,0g; 4,4 mmol) in 30 ml 10 %iger Salzsäure wird 2 h unter Rückfluß erhitzt. Das Lösungsmittel wird im Vakuum abgezogen und der Rückstand wird mit Tetrahydrofuran gewaschen.
Ausbeute: 0,22 g (23 % eines Diastereomerengemisches)
C₅H₉NO₄S x HCl (179.2 x 36.5)
Smp.: >250°C

## Patentansprüche

1. Verwendung von substituierten Tetrahydrothiophenen der allgemeinen Formel (I) 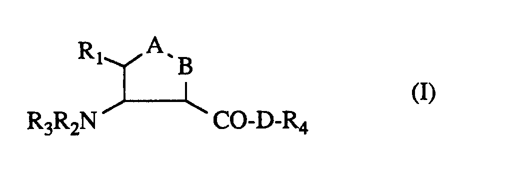 in welcher
A und B stets verschieden sind und
für ein Schwefelatom oder für die Gruppe der Formel -SO, -SO₂ oder -CHR⁵ stehen,
worin
R⁵ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
R¹ für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR⁶R⁷ substituiert ist,
worin
R⁶ und R⁷ gleich oder verschieden sind und Wasserstoff, Phenyl oder geradkettiges oder verzweigtes Alkyl mit bis zu 6 Kohlenstoffatomen bedeuten,
R² für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen, oder
für Benzoyl steht, das gegebenenfalls wie oben beschrieben substituiert ist, oder
für eine Gruppe der Formel -SO₂R⁸ steht,
worin
R⁸ geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR⁶R⁷ substitituiert sind,
worin
R⁶ und R⁷ die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR⁶R⁷ oder -SO₂R⁸ substituiert ist,
worin
R⁶, R⁷ und R⁸ die oben angegebene Bedeutung haben,
R³ für Wasserstoff oder für geradkettiges oder verzweigtes Alkyl mit bis zu 8 Kohlenstoffatomen steht, das gegebenenfalls durch Phenyl substituiert ist,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder Schwefelatom oder für die 〉NH-Gruppe steht,
R⁴ für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR⁶R⁷ oder -SO₂R⁸ substituiert sind,
worin
R⁶, R⁷ und R⁸ die oben angegebene Bedeutung haben,
oder für den Fall, daß D für die 〉NH-Gruppe steht,
R⁴ für die Gruppe der Formel -SO₂R⁸ steht,
worin
R⁸ die oben angegebene Bedeutung hat,
zur Bekämpfung von Krankheiten.

2. Verwendung von substituierten Tetrahydrothiophenen der allgemeinen Formel (I) gemäß Anspruch 1, in welcher
A und B stets verschieden sind und
für ein Schwefelatom oder für die Gruppe der Formel -SO, -SO₂ oder -CHR⁵ stehen,
worin
R⁵ Wasserstoff oder geradkettiges oder verzweigtes Alkyl mit bis zu 6 Kohlenstoffatomen bedeutet, das gegebenenfalls durch Halogen, Hydroxy, oder durch geradkettiges oder verzweigtes Alkoxy oder Alkoxycarbonyl mit jeweils bis zu 4 Kohlenstoffatomen substituiert ist,
R¹ für Wasserstoff oder für geradkettiges oder verzweigtes Alkyl mit bis zu 6 Kohlenstoffatomen steht, das gegebenenfalls durch Halogen, Hydroxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ substituiert ist,
worin
R⁶ und R⁷ gleich oder verschieden sind und Wasserstoff oder geradkettiges oder verzweigtes Alkyl mit bis zu 4 Kohlenstoffatomen bedeuten,
R² für Wasserstoff oder
für geradkettiges oder verzweigtes Alkyl mit bis zu 6 Kohlenstoffatomen steht, das gegebenenfalls durch Hydroxy, Formyl oder durch geradkettiges oder verzweigtes Acyl mit bis zu 4 Kohlenstoffatomen oder durch Phenyl oder Benzoyl substiutiert ist, die gegebenenfalls durch Halogen, Nitro, Cyano, oder durch geradkettiges oder verzweigtes Alkyl mit bis zu 4 Kohlenstoffatomen substituiert sind,
oder
für geradkettiges oder verzweigtes Acyl mit bis zu 6 Kohlenstoffatomen oder für Benzoyl steht, das gegebenenfalls wie oben beschrieben substituiert ist, oder
für eine Gruppe der Formel -SO₂R⁸ steht,
worin
R⁸ geradkettiges oder verzweigtes Alkyl mit bis zu 6 Kohlenstoffatomen, Phenyl oder Benzyl bedeutet, wobei letztere gegebenenfalls bis zu 2-fach gleich oder verschieden durch Halogen, Hydroxy, Nitro, Cyano, Trifluormethyl, Trifluormethoxy oder durch geradkettiges oder verzweigtes Alkyl oder Alkoxy mit jeweils bis zu 4 Kohlenstoffatomen oder durch die oben aufgeführte Gruppe der Formel -NR⁶R⁷ substituiert sind,
worin
R⁶ und R⁷ die oben angegebene Bedeutung haben,
für Phenyl steht, das gegebenenfalls bis zu 2-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 4 Kohlenstoffatomen oder durch eine Gruppe der Formel -NR⁶R⁷ oder -SO₂R⁸ substituiert ist,
worin
R⁶, R⁷ und R⁸ die oben angegebene Bedeutung haben,
R³ für Wasserstoff oder für geradkettiges oder verzweigtes Alkyl mit bis zu 6 Kohlenstoffatomen oder Benzyl steht,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder Schwefelatom oder für die
〉NH-Gruppe steht,
R⁴ für Wasserstoff oder für geradkettiges oder verzweigtes Alkyl mit bis zu 6 Kohlenstoffatomen oder Phenyl steht, wobei letztere gegebenenfalls bis zu 2-fach gleich oder verschieden durch Hydroxy, Halogen, Nitro, Cyano, Trifluormethyl, Trifluormethoxy, durch geradkettiges oder verzweigtes Alkoxy, Acyl oder Alkoxycarbonyl mit jeweils bis zu 4 Kohlenstoffatomen oder durch eine Gruppe der Formel -NR⁶R⁷ oder -SO₂R⁸ substituiert sind,
worin
R⁶, R⁷ und R⁸ die oben angegebene Bedeutung haben,
oder für den Fall, daß D für die 〉NH-Gruppe steht,
R⁴ für die Gruppe der Formel -SO₂R⁸ steht,
worin
R⁸ die oben angegebene Bedeutung hat,
zur Bekämpfung von Krankheiten.

3. Verwendung von substituierten Tetrahydrothiophenen der allgemeinen Formel (I) gemäß Anspruch 1, in welcher
A und B stets verschieden sind und
für ein Schwefelatom oder für die Gruppe der Formel -SO, -SO₂ oder -CHR⁵ stehen,
worin
R⁵ Wasserstoff oder geradkettiges oder verzweigtes Alkyl mit bis zu 4 Kohlenstoffatomen bedeutet,
R¹ für Wasserstoff oder
für geradkettiges oder verzweigtes Alkyl mit bis zu 4 Kohlenstoffatomen steht,
R² für Wasserstoff oder
für geradkettiges oder verzweigtes Alkyl mit bis zu 4 Kohlenstoffatomen steht, oder
für geradkettiges oder verzweigtes Acyl mit bis zu 4 Kohlenstoffatomen oder
für eine Gruppe der Formel -SO₂R⁸ steht,
worin
R⁸ geradkettiges oder verzweigtes Alkyl mit bis zu 4 Kohlenstoffatomen, Phenyl oder Benzyl bedeutet, wobei letztere gegebenenfalls durch Hydroxy, Fluor, Chlor, Brom, Nitro, Cyano, Methyl, Ethyl oder Methoxy substituiert sind,
R³ für Wasserstoff oder
für geradkettiges oder verzweigtes Alkyl mit bis zu 4 Kohlenstoffatomen steht,
oder
R² und R³ gemeinsam für den Rest der Formel =CHR^{5'} stehen,
worin
R^{5'} die oben angegebene Bedeutung von R⁵ hat und mit dieser gleich oder verschieden ist,
D für ein Sauerstoff- oder ein Schwefelatom oder für die 〉NH-Gruppe steht,
R⁴ für Wasserstoff oder für geradkettiges oder verzweigtes Alkyl mit bis zu 4 Kohlenstoffatomen oder Phenyl steht, wobei letztere gegebenenfalls durch Fluor, Chlor, Brom, Nitro, Cyano, Methoxy, Ethoxy oder durch eine Gruppe der Formel -NR⁶R⁷ oder -SO₂R⁸ substituiert sind,
worin
R⁶und R⁷ gleich oder verschieden sind und Wasserstoff, Methyl oder Ethyl bedeuten
und
R⁸ die oben angegebene Bedeutung hat,
oder im Fall, daß D für die 〉NH-Gruppe steht,
R⁴ für die Gruppe der Formel -SO₂R⁸ steht,
worin
R⁸ die oben angegebene Bedeutung hat,
zur Bekämpfung von Krankheiten.

4. Substituierte Tetrahydrothiophene der allgemeinen Formel (Ia) 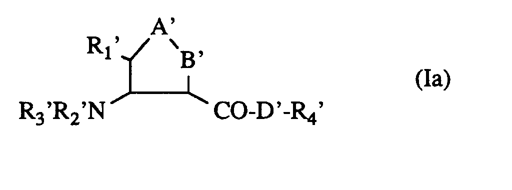 in welcher
A' und B' stets verschieden sind und
für ein Schwefelatom oder für die Gruppe der Formel -SO, -SO₂ oder -CHR⁹ stehen,
worin
R⁹ Wasserstoff oder geradkettiges oder verzweigtes Alkyl mit bis zu 8 Kohlenstoffatomen bedeutet, das gegebenenfalls durch Halogen, Hydroxy, Phenyl, Carboxy oder durch geradkettiges oder verzweigtes Alkoxy oder Alkoxycarbonyl mit jeweils bis zu 6 Kohlenstoffatomen substituiert ist,
R^{1'} für Wasserstoff oder für geradkettiges oder verzweigtes Alkyl mit bis zu 8 Kohlenstoffatomen steht, das gegebenenfalls 1- bis 2-fach gleich oder verschieden durch Halogen, Hydroxy, Phenyl, Carboxy oder durch geradkettiges oder verzweigtes Alkoxy, Acyl oder Alkoxycarbonyl mit jeweils bis zu 6 Kohlenstoffatomen oder durch eine Gruppe der Formel -NR^{6'}R^{7'} substituiert ist,
worin
R^{6'} und R^{7'} gleich oder verschieden sind und Wasserstoff, Phenyl oder geradkettiges oder verzweigtes Alkyl mit bis zu 6 Kohlenstoffatomen bedeuten,
R^{2'} für Wasserstoff oder
für geradkettiges oder verzweigtes Alkyl mit bis zu 8 Kohlenstoffatomen steht, das gegebenenfalls 1- bis 2-fach gleich oder verschieden durch Hydroxy, Formyl oder durch geradkettiges oder verzweigtes Acyl mit bis zu 6 Kohlenstoffatomen oder durch Phenyl oder Benzoyl substituiert ist, die gegebenenfalls bis zu 2-fach gleich oder verschieden durch Halogen, Nitro, Cyano, oder durch geradkettiges oder verzweigtes Alkyl mit bis zu 6 Kohlenstoffatomen substituiert sind,
oder
für geradkettiges oder verzweigtes Acyl mit bis zu 8 Kohlenstoffatomen, oder
für Benzoyl steht, das gegebenenfalls wie oben beschrieben substituiert ist, oder
für eine Gruppe der Formel -SO₂R^{8'} steht,
worin
R^{8'} geradkettiges oder verzweigtes Alkyl mit bis zu 8 Kohlenstoffatomen, Benzyl oder Phenyl bedeutet, wobei letztere gegebenenfalls bis zu 3-fach gleich oder verschieden durch Halogen, Hydroxy, Nitro, Cyano, Trifluormethyl, Trifluormethoxy oder durch geradkettiges oder verzweigtes Alkyl, Alkoxy oder Alkoxycarbonyl mit jeweils bis zu 6 Kohlenstoffatomen, Carboxy oder durch die oben aufgeführte Gruppe -NR^{6'}R^{7'} substitituiert sind,
worin
R^{6'} und R^{7'} die oben angegebene Bedeutung haben,
für Phenyl steht, das gegebenenfalls bis zu 3-fach gleich oder verschieden durch Halogen, Hydroxy, Nitro, Trifluormethyl, Trifluormethoxy, geradkettiges oder verzweigtes Alkyl, Acyl, Alkoxy oder Alkoxycarbonyl mit jeweils bis zu 6 Kohlenstoffatomen oder durch eine Gruppe der Formel -NR^{6'}R^{7'} oder -SO₂R^{8'} substituiert ist,
worin
R^{6'}, R^{7'} und R^{8'} die oben angegebene Bedeutung haben,
R^{3'} für Wasserstoff oder für geradkettiges oder verzweigtes Alkyl mit bis zu 8 Kohlenstoffatomen steht, das gegebenenfalls durch Phenyl substituiert ist,
oder
R^{2'} und R^{3'} gemeinsam für den Rest der Formel =CHR^{9'} stehen,
worin
R^{9'} die oben angegebene Bedeutung von R⁹ hat und mit dieser gleich oder verschieden ist,
D' für ein Sauerstoff- oder Schwefelatom oder für die 〉NH-Gruppe steht,
R^{4'} für Wasserstoff oder für geradkettiges oder verzweigtes Alkyl mit bis zu 8 Kohlenstoffatomen oder Phenyl steht, wobei letztere gegebenenfalls bis zu 3-fach gleich oder verschieden durch Hydroxy, Halogen, Nitro, Cyano, Carboxy, Trifluormethyl, Trifluormethoxy, durch geradkettiges oder verzweigtes Alkoxy, im Fall von Phenyl auch durch Alkyl, Acyl oder Alkoxycarbonyl mit jeweils bis zu 6 Kohlenstoffatomen oder durch eine Gruppe der Formel -NR^{6'}R^{7'} oder -SO₂R^{8'} substituiert sind,
worin
R^{6'}, R^{7'} und R^{8'} die oben angegebene Bedeutung haben,
oder für den Fall, daß D für die 〉NH-Gruppe steht,
R^{4'} für die Gruppe der Formel -SO₂R^{8'} steht,
worin
R^{8'} die oben angegebene Bedeutung hat,
mit der Maßgabe, daß wenn A' für ein Schwefelatom, B' für die -CH₂-Gruppe, D' für ein Sauerstoffatom und R^{1'}, R^{2'}, R^{3'} und R^{4'} für Wasserstoff stehen, die beiden Substituenten -NR^{2'}R^{3'} und -CO-D'-R^{4'} nicht beide in trans-Stellung vorliegen dürfen.

5. Verfahren zur Herstellung der Verbindungen der allgemeinen Formel (I) gemäß Anspruch 1, dadurch gekennzeichnet, daß man
[A] im Fall, daß A und A' jeweils entsprechend für die -CHR⁵ oder -CHR⁹-Gruppe und B bzw. B' für ein Schwefelatom stehen,
Verbindungen der allgemeinen Formel (II) 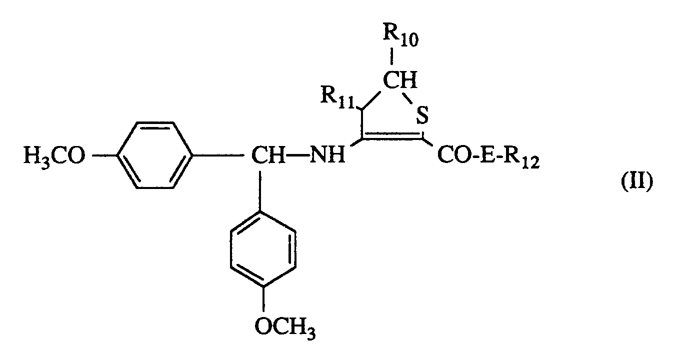 in welcher
R¹⁰ die oben angegebene Bedeutung von R⁵ und R⁹ umfaßt,
R¹¹ den oben angegebenen Bedeutungsumfang von R¹ und R^{1'} umfaßt,
E für ein Sauerstoffatom steht
und
R¹² für C₁-C₆-Alkyl steht,
zunächst mit Natriumcyanoborhydrid in inerten Lösemittel,
in die Verbindungen der allgemeinen Formel (III) 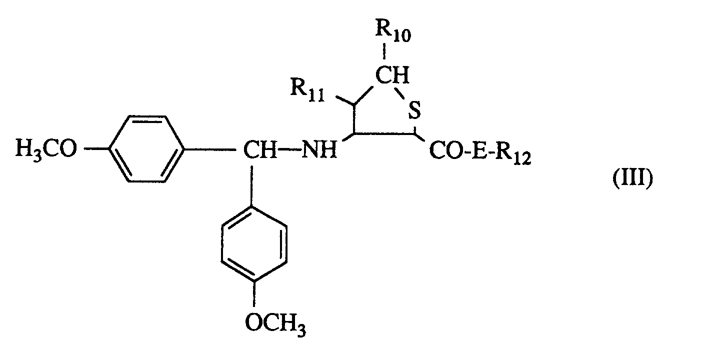 in welcher
R¹⁰, R¹¹, R¹² und E die oben angegebene Bedeutung haben
überführt,
und anschließend mit Säuren und Wasser, vorzugsweise Essigsäure, die Aminfunktion deblockiert
oder
[B] im Fall, daß A und A' für ein Schwefelfatom und B bzw. B' für die -CHR⁵ oder -CHR⁹-Gruppe stehen,
Verbindungen der allgemeinen Formel (IV) 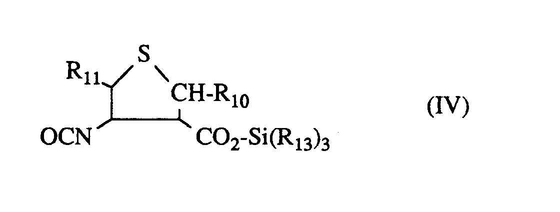 in welcher
R¹⁰ und R¹¹ die oben angegebene Bedeutung haben
und
R¹³ für einen C₁-C₃-Alkylrest steht,
in Ethern, vorzugsweise Diethylether, in Anwesenheit von Wasser zunächst zu den Verbindungen der allgemeinen Formel (V) 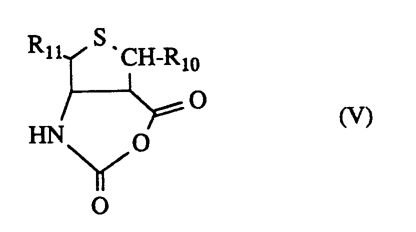 in welcher
R¹⁰ und R¹¹ die oben angegebene Bedeutung haben,
umsetzt
und in einem nächsten Schritt mit Säuren, vorzugsweise Salzsäure und nachfolgend Propylenoxid, unter Ringöffnung, in die Verbindungen der allgemeinen Formel (Ib) 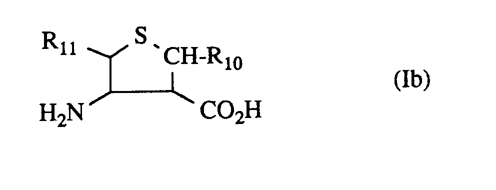 in welcher
R¹⁰ und R¹¹ die oben angegebene Bedeutung haben,
überführt,
oder
[C] im Fall, daß B und B' für die -SO oder -SO₂-Gruppe stehen,
Verbindungen der allgemeinen Formel (Ic) 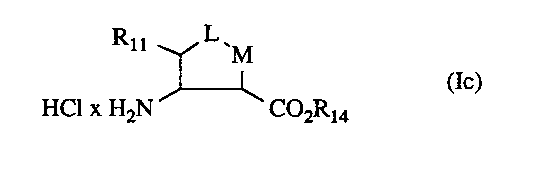 in welcher
R¹⁰ und R¹¹ die oben angegebene Bedeutung haben,
R¹⁴ für C₁-C₄-Alkyl steht
und
L und M verschieden sind und für ein Schwefelatom oder für die -CHR¹⁰-Gruppe stehen,
zunächst in inerten Lösemitteln, in Anwesenheit einer Base, vorzugsweise Triethylamin, nach Blockierung der freien Aminfunktion zu den Verbindungen der allgemeinen Formel (VI) 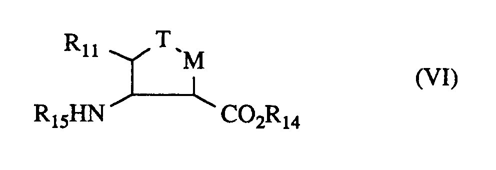 in welcher
M und T verschieden sind, und M die oben angegebene Bedeutung hat und T für die -SO- oder -SO₂-Gruppe steht,
R¹⁰, R¹¹ und R¹⁴ die oben angegebene Bedeutung haben
und
R¹⁵ für eine literaturbekannte Aminoschutzgruppe, vorzugsweise tert.Butoxycarbonyl (BOC) steht
mit Oxidationsmitteln, vorzugsweise m-Chlorperbenzoesäure oxidiert und anschließend nach üblicher Methode, vorzugsweise mit Säuren, die Schutzgruppe abspaltet,
und im Fall der Säuren [(I), (Ia) D, E=O, R⁴/R^{4'}=H] gegebenenfalls die entsprechenden Ester verseift,
und im Fall der für D/E und R⁴/R^{4'} oben aufgeführten anderen Definitionen, ebenfalls nach üblichen Verfahren, wie beispielsweise Amidierung, Sulfonierung oder Sulfoamidierung, gegebenenfalls in Anwesenheit von Hilfsstoffen wie Katalysatoren und Dehydratisierungsmitteln, ausgehend von den entsprechenden Carbonsäuren, gegebenenfalls unter vorgeschalteter Aktivierung, derivatisiert.

6. Arzneimittel enthaltend Verbindungen der allgemeinen Formel (I) gemäß den Ansprüchen 1 bis 4.

7. Verwendung von Verbindungen der allgemeinen Formel (I) gemäß den Ansprüchen 1 bis 4 zur Herstellung von Arzneimitteln für die Human- oder Veterinärmedizin.
